# EUROPEAN PATENT APPLICATION

(11) **EP 2 859 907 A1**
(43) Date of publication of application: **15.04.2015**
(21) Application number: 14180645.5
(22) Date of filing: 12.08.2014
(51) Int. Cl.: A61M 25/01, A61M 29/00, A61M 25/00, A61M 25/06

(54) **Steerable medical devices**

(30) Priority: 10.10.2013 US 201361889201 P; 08.08.2014 US 201414455157
(71) Applicant: Oscor Inc., Palm Harbor, FL 34683 (US)
(72) Inventor: Osypka, Thomas P., Palm Harbor, Florida 34685 (US); Damascelli, Bruno, 20133 Milano (IT)
(74) Representative: Hoefer & Partner

(57) **Abstract**

A steerable medical device (100) includes a sheath having (110) a preformed non-linear configuration, a deflectable distal end portion (114), and at least one lateral passage (144) configured to accommodate at least one steering wire (134). The device further includes a steering handle (116) operatively associated with a proximal end portion of the sheath and having an actuation mechanism operatively connected to the at least one steering wire accommodated within the at least one lateral passages of the sheath for steering the deflectable distal end portion of the sheath in at least one direction.

## Description

### CROSS-REFERENCE TO RELATED APPLICATIONS

This application claims the benefit of and priority to U.S. Provisional Application No. 61/889,201, filed October 10, 2013, the content of which is incorporated by reference herein in its entirety.

### BACKGROUND OF THE INVENTION

### 1. Field of the Invention

The subject invention is directed to steerable medical devices such as guiding sheaths and handles therefor, which are adapted and configured for the introduction and placement of diagnostic and therapeutic devices into the human vasculature.

### 2. Description of Related Art

Guiding catheters ("sheaths") are commonly used to introduce balloon catheters and stents into the vascular system (e.g., for percutaneous transvascular coronary angioplasty), to introduce cardiac pacing leads into the coronary sinus (e.g., for left ventricular pacing and cardiac resynchronization procedures), or to introduce radiofrequency ablation catheters into the left atrium (e.g., for treatment of atrial fibrillation) into the renal artery for renal denervation procedures.

Guiding sheaths typically come in French sizes ranging from 4F all the way to 12F, and in some cases even 18F. Sheaths generally include an inner lumen that extends from the proximal portion of the device to the distal tip section of the device. The inner lumen often has a polytetrafluoroethylene (PTFE) liner to make the insertion of a device therethrough as easy and as smooth as possible.

In some cases, guiding sheaths can have pre-shaped distal tips with a curved configuration designed to fit the anatomy of the vascular system and/or to help find the correct access into a particular vein or artery once introduced. Such a pre-shaped configuration can allow the placement of the guiding sheath into the desired location of the vascular system, which then allows the introduction of more complex medical instruments and devices for performing a medical procedure.

However, due to the variation of the vascular system from patient to patient, it is very difficult to place a sheath having a fixed curvature into a desired location. As a result, several guiding catheters with different curved shapes must be used to get to a desired location. This adds cost and time to the procedure, with the additional risk that some procedures may have to aborted due to unsuccessful positioning of the sheath.

It would be beneficial to provide a system for accurately and efficiently placing a guiding sheath having a preformed curvature into the vascular system of a patient.

### SUMMARY

In at least one aspect of this disclosure, a steerable medical device includes a sheath having a preformed non-linear configuration, a deflectable distal end portion, and at least one lateral passage configured to accommodate at least one steering wire. The device further includes a steering handle operatively associated with a proximal end portion of the sheath and having an actuation mechanism operatively connected to the at least one steering wire accommodated within the at least one lateral passages of the sheath for steering the deflectable distal end portion of the sheath in at least one direction. The sheath can include a central lumen for facilitating the introduction of a medical device into the vasculature of a patient.

The preformed non-linear configuration of the sheath can be a configuration having at least one curved section. The preformed non-linear configuration of the sheath can be a configuration including an undulating shape such that the sheath includes at least two curves. In some embodiments, the preformed non-linear configuration of the sheath can be a configuration having a spiral configuration such that the sheath spirals about a longitudinal axis of the device.

The deflectable distal end portion of the sheath can have an operative length of between about 2 cm and about 4 cm for steering the distal end portion of the sheath. The deflectable distal end portion of the sheath can be deflectable in at least two directions by at least about 45 degrees relative to a longitudinal axis of the sheath.

The steering handle can be configured for effectuating bi-directional steering of the sheath. The actuation mechanism can include a drive nut threadably coupled to a worm coil such that rotation of the drive nut causes axial translation of the worm coil within the steering handle, wherein the worm coil is operably connected to one steering wire for mono-directional steering of the distal end portion of the sheath. A manually rotatable torque handle can be operatively connected to the drive nut.

A hemostatic seal can be operatively associated with the proximal end portion of the sheath in fluid communication with the central lumen. The sheath can include an outer diameter size ranging from about 4F to about 18F.

The proximal end portion of the sheath can extend through the steering handle to a proximal end thereof. The sheath can include a reinforced section. An infusion port can be operatively associated with the proximal end portion of the sheath.

The device can further include a flexible dilator dimensioned for introduction through the central lumen of the sheath and having an axial passage extending therethrough for accommodating a flexible guide wire. The device can further include a flexible guide wire for introduction through the axial passage of the flexible dilator.

A kit for placing a surgical device in the vasculature of patient can include an enclosure, a steerable medical device as disclosed herein, and a dilator disposed within the enclosure configured to be inserted into the sheath. The kit can further include a guide wire disposed within the enclosure.

### BRIEF DESCRIPTION OF THE DRAWINGS

So that those skilled in the art to which the subject invention appertains will readily understand how to make and use the subject invention without undue experimentation, reference may be had to the figures, wherein:
Fig. 1A is an illustration of an embodiment of a steerable preformed guiding sheath having a preformed distal tip in accordance with this disclosure, operative with a bi-directional steering handle and adapted for the introduction and placement of diagnostic and therapeutic devices into the human vasculature;
Fig. 1B is a cross-sectional view of the handle portion of the steerable guiding sheath, with reference to Fig. 1A;
Fig. 1C is a cross-sectional view of an embodiment of the sheath shown in Fig. 1B;
Fig. 1D is a depiction of the steerable sheath of Fig. 1A shown having the distal end portion of the preformed sheath in a first deflected position;
Fig. IE is a depiction of the steerable sheath of Fig. 1A shown having the distal end portion of the preformed sheath in a second deflected position;
Fig. 1F is a depiction of the bi-directional steerable guiding sheath of Fig. 1A shown having the distal end portion of the sheath in a straight condition with a dilator disposed therein;
Fig. 2A is an illustration of an embodiment of a steerable preformed guiding sheath having a preformed distal tip in accordance with this disclosure, operative with a mono-directional steering handle and in a straightened configuration having a dilator inserted therein;
Fig. 2B is a depiction of the steerable sheath of Fig. 2A shown having the distal end portion of the preformed sheath in a non-steered configuration;
Fig. 2C is a depiction of the steerable sheath of Fig. 2A shown having the distal end portion of the preformed sheath in a deflected position;
Fig. 2D is a partial cross-sectional view of the mono-directional handle of the steerable preformed sheath of Fig. 1A; and
Fig. 3 is an in-situ view of an embodiment of this disclosure disposed within a renal artery during a medical procedure.

### ENABLING DESCRIPTION OF THE INVENTION

Referring now to the drawings wherein like reference numerals identify similar structural features or elements of the disclosed devices, embodiments of this disclosure are directed to preformed steerable guiding catheters.

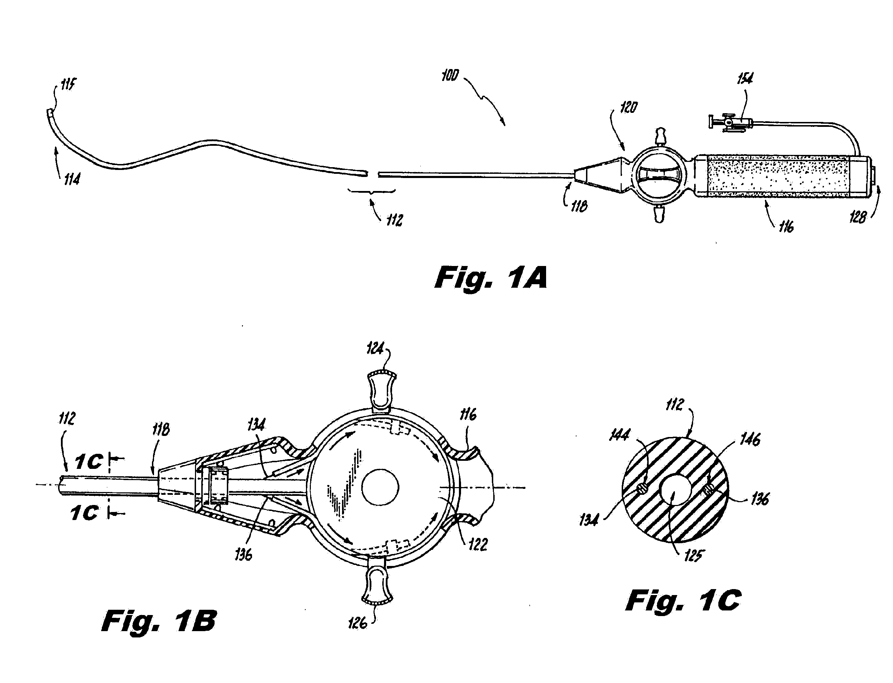

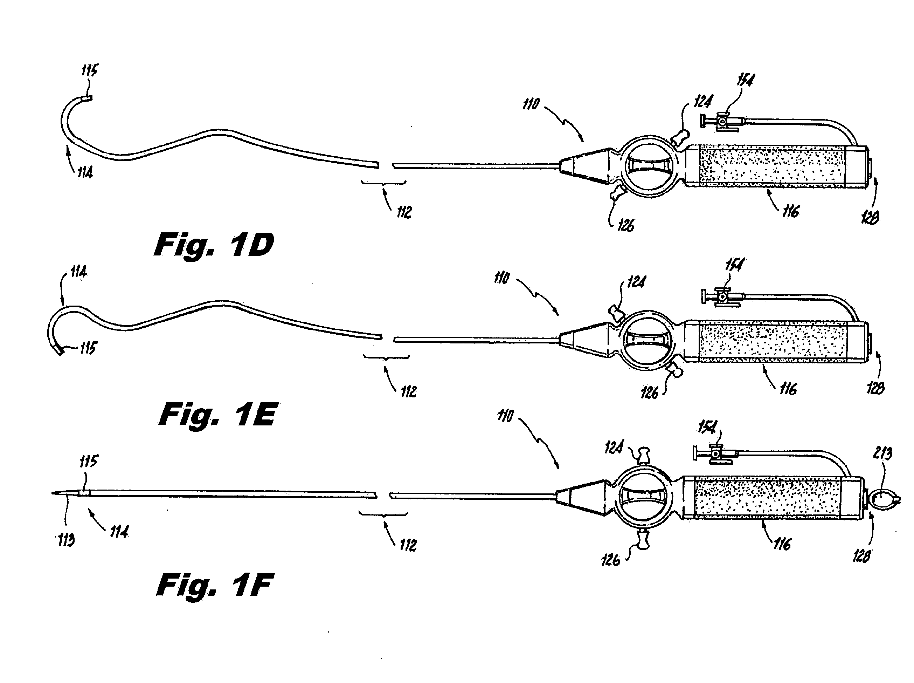

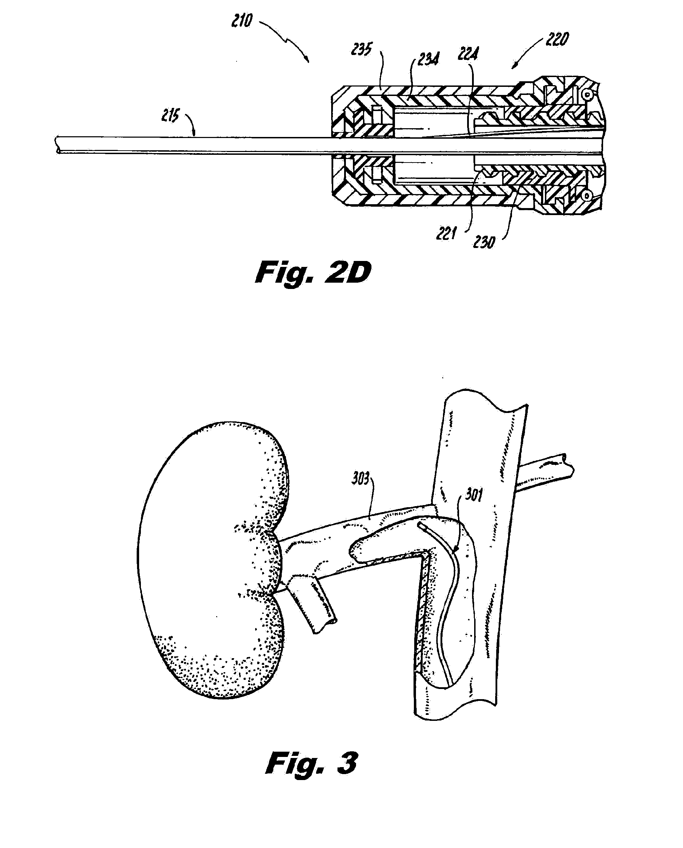

In at least one aspect of this disclosure, referring generally to Figs. 1A-1F, a preformed steerable guiding sheath 110 can include an elongated sheath body 112 having a preformed, deflectable distal end portion 114. The sheath 312 can have a fixed or otherwise preformed non-linear configuration to enable correct placement of the distal end portion 114 of the sheath body 112 in an anatomical structure (e.g., a renal artery or other suitable location) of a patient, as illustrated in Fig. 3.

In at least some embodiments, the sheath body 112 has at least one curved section. In embodiments, the preformed sheath body 112 can include a curved or undulating configuration with at least two curved sections. However, the preformed shape of the sheath body 112 can vary depending upon the surgical procedure and/or the anatomy of the patient. Any other suitable shape or design for the preformed sheath is contemplated herein.

The sheath body 112 can include any suitable cross-sectional design, e.g., a single biocompatible plastic layer as shown in Fig. 1C, or any other suitable multilayer structure/material selection (e.g., including any suitable reinforcement along at least a portion of sheath body 112). The sheath body 112 can have a hydrophobic coating or any other suitable and/or desired coating. In some embodiments, the sheath body 112 can be formed from a hydrophobic material.

The sheath body 112 can also have any suitable outer diameter size for a desired use. In some embodiments, the outer diameter size of the sheath body 112 ranges from about 4F to about 18F. In some embodiments, the outer diameter of the sheath body 112 is about 5F.

The sheath body 112 can also include a soft atraumatic tip portion 115 disposed on the distal end thereof. In some embodiments, the tip portion 115 can include one or more side holes (not shown) in fluid communication with an infusion port 154 (e.g., including a conventional leur fitting) associated with the steering handle 116.

The distal end portion 114 can also include a suitable radiopaque marker (not shown) for locating the distal end portion 114 in situ to enable the visual guidance of the sheath 110 through the vascular system of a patient using a suitable imaging system.

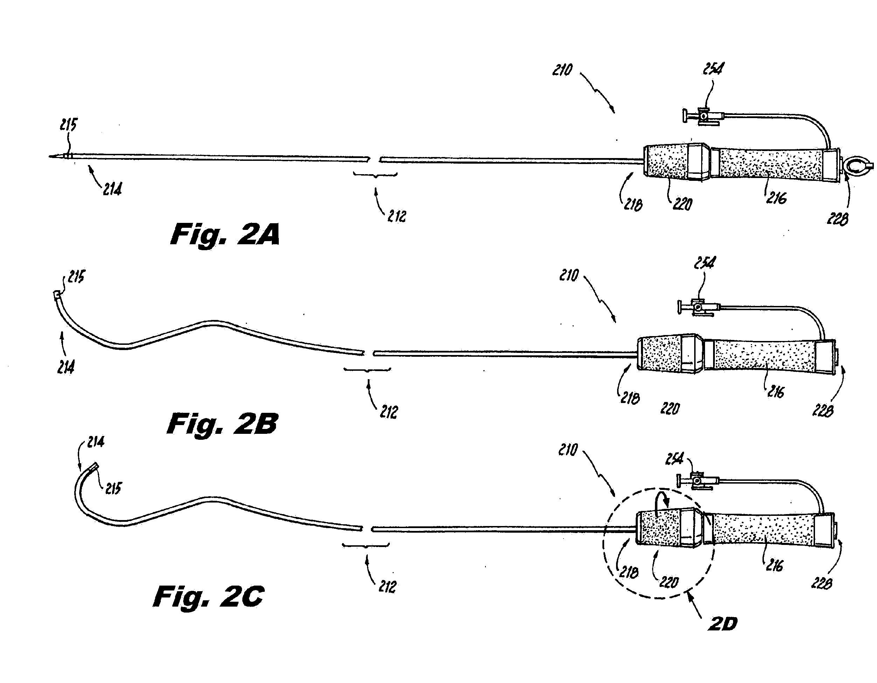

The sheath body 112 can include and/or define a central lumen 125 and at least one lateral passage (e.g., passage 144) to accommodate one or more steering wires (e.g., steering wire 134). As such, the sheath 110 can be configured for mono-directional, bi-directional, or multi-directional steering. Any suitable steering handle (e.g., the bi-directional handle of Fig. 1A; the mono-directional handle of Fig. 2A) is contemplated herein to operatively associate with the preformed sheath as disclosed herein. To effect deflection of the distal end portion 114, the distal end portion 114 can include and anchor member (not shown) disposed therein configured to anchor the one or more steering wires 134, 136 to the distal end portion 114 such that the anchor member does not move relative to the distal tip when pulled on by the steering wires 134, 136.

In some embodiments, the deflectable distal end portion 114 of the preformed sheath body 112 can include an operative steering length of between about 2 cm to about 4 cm. However, it is contemplated that the sheath body 112 can bend at any suitable point along the length thereof such that the operative length is selected to be any suitable portion thereof. It is also contemplated that the degree of deflection can be any suitable deflection, e.g., deflectable to anywhere between about 0 degrees to about 180 degrees relative to the longitudinal axis of the sheath body 112 in any suitable direction. In some embodiments, the distal end portion 114 can deflect up to about 45 degrees.

Referring specifically, Figs. 1A-1F, the sheath body 112 is shown operative with a bi-directional embodiment of a handle. In such an embodiment, as shown in Fig. 1C, a pair of diametrically opposed lateral passages 144, 146 to accommodate a corresponding pair of steering wires 134, 136 is disposed within the sheath body 112. While the embodiment of Fig. 1C is shown as having two lateral passages 144, 146 and two steering wires 134, 136, any suitable number of passages and/or cables are contemplated herein, e.g., one, two, three, four, or more.

The proximal end portion 118 of the sheath body 112 can extend through the steering handle 116 to a proximal end thereof. A hemostatic seal 128 can be operatively associated with the proximal end portion 118 of the sheath body 112 such that the hemostatic seal 128 is in fluid communication with the central lumen 125 thereof. In at least some embodiments, the hemostatic seal 128 can provide an effective seal for a guide wire of about 0.014 inches or any other suitable size.

Referring to Fig. 1B, the steering handle 116 includes an actuation mechanism 120 that is operatively connected to the pair of steering wires 134, 136 accommodated within the opposed lateral passages 144, 146 of the sheath body 112 for steering the deflectable distal end portion 114 of the sheath body 112. The actuation mechanism 120 of the steering handle 116 can include a central hub 122 connected to the actuators 124, 126 and to the steering wires 134, 136.

Referring to Figs. 1D-1F the preformed sheath body 112 is shown in a first deflected position (Fig. 1D) and a second deflected position (Fig. IE), and being transformed to an non-steered and straight configuration (Fig. 1F) with a dilator 113 inserted therein. In use, manipulation of the actuators 124 and 126 in clockwise and counter-clockwise directions causes the corresponding movement of the central hub and steering wires 134 and 136. This results in the bi-directional deflection of the distal end portion 114 of the sheath body 112. It is contemplated that clockwise actuator motion can lead to a counter-clockwise tip deflection, and vice versa. The actuation mechanism 120 controls the orientation of the distal end portion of the sheath and can be designed to have any suitable maneuverability (e.g., 180° dual deflection maneuverability).

While the above described embodiment is shown having a bi-directional steering handle associated therewith of a certain design, it is contemplated that any suitable bi-directional handle can be used associated with the preformed sheath body 112.

In at least one aspect of this disclosure, referring now to Figs. 2A-2D, another embodiment of a steerable preformed sheath 210 is shown having a differing handle assembly 216 than the above described embodiment in Fig. 1A. The steerable preformed sheath 210 includes an elongated sheath body 212 having a deflectable distal end portion 214, and is otherwise similar to sheath body 112 described above (e.g., including an atraumatic tip 215, a hemostatic seal 228).

As shown in this embodiment, the sheath body 212 is operative to steer in one direction and is connected to a mono-directional handle assembly 216. The handle assembly 216 includes a manually or automatically operable actuation mechanism 220. The actuation mechanism 220 can be operatively connected to a steering wire 224 for steering the distal end portion 214 similar to described above with respect to the embodiment of Fig. 1A.

As best seen in Fig. 2D, the actuation mechanism 220 can include a drive nut 230 that is threadably coupled to a worm coil 221. Rotation of the drive nut 230 causes axial translation of the worm coil 221 within the handle assembly 216. The worm coil 221 can be connected to the steering wire 224 such that proximal advancement of the worm coil 221 pulls on the steering wire 224 causing the distal end portion 214 to deflect in a direction.

The actuation mechanism 220 further includes a manually rotatable torque ring 234 that is operatively connected to the drive nut 230 and configured to be rotated by a user. The torque ring 234 can be positioned adjacent a stationary torque grip 235, thereby enabling a user to maintain a firm grip on the device 210 while rotating the torque ring 234 to achieve the mono-directional deflection of the distal end portion 214 of the sheath 212.

Referring to Fig. 2A, the sheath 210 is shown in a straightened, non-steered configuration with a dilator 213 inserted therein. Fig. 2B shows the dilator 213 removed therefrom and the sheath body 212 reverting to the preformed shape thereof. Fig. 2C shows the preformed distal end portion 214 being steered to about a 180 degree position relative to the straightened position.

While the embodiments herein show the sheaths 110, 210 being steered only without the dilator 113, 213 inserted therein, it is contemplated that the sheaths 110,210 can also be steered in the straightened configuration with the dilator 113, 213 inserted therein.

Referring to Fig. 3, using a steerable preformed sheath 301 as described in the above embodiments can allow easier entrance to, e.g., the renal artery 303 from the formal side, or improve access to other vasculature or anatomical structures. In addition, the ability to accurately steer the deflectable distal end portion of the preformed sheath body with a high degree of precision enables the user to accommodate for variable anatomy and accurately introduce a medical device, such as, for example, a renal ablation catheter into the renal artery.

The devices, methods, and systems of the present disclosure, as described above and shown in the drawings, provide for steerable medical devices with superior properties including advanced directional and precision control. While the apparatus and methods of the subject disclosure have been shown and described with reference to embodiments, those skilled in the art will readily appreciate that changes and/or modifications may be made thereto without departing from the spirit and scope of the subject disclosure.

## Claims

1. A steerable medical device, comprising:
a) a sheath including a preformed non-linear configuration, the sheath having a deflectable distal end portion, and at least one lateral passage configured to accommodate at least one steering wire; and
b) a steering handle operatively associated with a proximal end portion of the sheath and having an actuation mechanism operatively connected to the at least one steering wire accommodated within the at least one lateral passages of the sheath for steering the deflectable distal end portion of the sheath in at least one direction.

2. The steerable device of claim 1, wherein the sheath includes a central lumen for facilitating the introduction of a medical device into the vasculature of a patient.

3. The steerable device of claim 1 or 2, wherein the preformed non-linear configuration of the sheath is a configuration having at least one curved section, or wherein the preformed non-linear configuration of the sheath is a configuration including an undulating shape such that the sheath includes at least two curves.

4. The steerable device of claim 1, wherein the preformed non-linear configuration of the sheath is a configuration having a spiral configuration such that the sheath spirals about a longitudinal axis of the device.

5. The steerable device of any of the preceeding claims, wherein the deflectable distal end portion of the sheath has an operative length of between about 2 cm and about 4 cm for steering the distal end portion of the sheath, and/or wherein the deflectable distal end portion of the sheath is deflectable in at least two directions by at least about 45 degrees relative to a longitudinal axis of the sheath.

6. The steerable device of claim 1, wherein the steering handle is configured for effectuating bi-directional steering of the sheath.

7. The steerable device of claim 6, wherein the actuation mechanism includes a drive nut threadably coupled to a worm coil such that rotation of the drive nut causes axial translation of the worm coil within the steering handle, wherein the worm coil is operably connected to one steering wire for mono-directional steering of the distal end portion of the sheath.

8. The steerable device of claim 7, wherein a manually rotatable torque handle is operatively connected to the drive nut.

9. The steerable device of claim 2, wherein a hemostatic seal is operatively associated with the proximal end portion of the sheath in fluid communication with the central lumen.

10. The steerable device of any of the preceeding claims, wherein the sheath has an outer diameter size ranging from about 4F to about 18F.

11. The steerable device of any of the preceeding claims, wherein the proximal end portion of the sheath extends through the steering handle to a proximal end thereof.

12. The steerable device of any of the preceeding claims, wherein the sheath includes a reinforced section.

13. The steerable device of any of the preceeding claims, further comprising a flexible dilator dimensioned for introduction through the central lumen of the sheath and having an axial passage extending therethrough for accommodating a flexible guide wire, and/or further comprising a flexible guide wire for introduction through the axial passage of the flexible dilator.

14. The steerable device of any of the preceeding claims, wherein an infusion port is operatively associated with the proximal end portion of the sheath.

15. A kit for placing a surgical device in the vasculature of patient, comprising:
a) an enclosure;
b) a steerable medical device disposed within the enclosure, wherein the steerable medical device includes:
i) a sheath including a preformed non-linear configuration, the sheath having a deflectable distal end portion, and at least one lateral passage configured to accommodate at least one steering wire; and
ii) a steering handle operatively associated with a proximal end portion of the sheath and having an actuation mechanism operatively connected to the at least one steering wire accommodated within the at least one lateral passages of the sheath for steering the deflectable distal end portion of the sheath in at least one direction; and
c) a dilator disposed within the enclosure configured to be inserted into the sheath.

16. The kit of claim 15, further comprising a guide wire disposed within the enclosure.
